(19) Europäisches Patentamt
European Patent Office
Office européen des brevets

(11) Numéro de publication: **0 162 781**
**B1**

# (12) FASCICULE DE BREVET EUROPÉEN

(45) Date de publication du fascicule du brevet: **02.08.89**

(21) Numéro de dépôt: **85400998.2**

(22) Date de dépôt: **21.05.85**

(51) Int. Cl.⁴: **A 61 K 47/00**, A 61 K 39/395, C 07 H 19/00, A 61 K 31/70, C 07 D 407/04, C 07 D 493/10, A 61 K 31/35 // (C07D407/04, 309:00, 307:00), (C07D493/10, 311:00, 307:00)

(54) **Conjugués associant par liaison covalente un ionophore carboxylique monovalent et une macromolécule, leur utilisation comme agents potentialisateurs des immunotoxines et les ionophores activés intermédiaires.**

(30) Priorité: **23.05.84 FR 8408073**
**13.07.84 FR 8411208**

(43) Date de publication de la demande: **27.11.85 Bulletin 85/48**

(45) Mention de la délivrance du brevet: **02.08.89 Bulletin 89/31**

(84) Etats contractants désignés: **AT BE CH DE FR GB IT LI LU NL SE**

(56) Documents cité:
**EP-A-0 024 189**
**EP-A-0 057 140**
**EP-A-0 080 401**
**EP-A-0 086 152**
**EP-A-0 116 911**

(73) Titulaire: **SANOFI, 40, Avenue George V, F-75008 Paris (FR)**

(72) Inventeur: **Jansen, Franz K., Chemin des Fresquets Assas, F-34160 Castries (FR)**
Inventeur: **Gros, Pierre, 18 rue des Muriers, F-34100 - Montpellier (FR)**

(74) Mandataire: **Gillard, Marie- Louise, Cabinet Beau de Loménie 55, Rue d'Amsterdam, F-75008 Paris (FR)**

**EP 0 162 781 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1989

**Description**

La présente invention concerne des nouveaux conjugués associant par liaison covalente un ionophore carboxylique monovalent et une macomolécule. Elle concerne également l'application desdits conjugués à titre de potentialisateurs des immunotoxines.

La présente invention concerne également les ionophores activés à titre d'intermédiaires de synthèse desdits conjugués.

Dans sa demande de brevet antérieure, déposée en France sous le n° 82/02 091, la demanderesse a mentionné la capacité que présentent les ionophores carboxyliques de potentialiser les immunotoxines.

Par immunotoxines, on désigne des substances anticancéreuses obtenues par couplage, par liaison covalente, d'une protéine cytotoxique (par exemple la chaîne A de la ricine) avec des anticorps ou fragments d'anticorps dirigés contre un antigène porté par une cellule à détruire.

De telles immunotoxines ont notamment été décrites par la demanderesse dans le brevet français n° 78/27 838 et son addition n° 79/24 655 et dans les demandes françaises n° 81/07 596 et n° 81/21 836. Les propriétés potentialisatrices des ionophores ont pu être utilisées avantageusement dans certains types de situations. En particulier, ils donnent des résultats favorables à chaque fois qu'une immunotoxine est utilisée en tant qu'agent cytotoxique sélectif in vitro pour détruire les cellules cibles. C'est notamment le cas lorsque l'immunotoxine est utilisée comme agent cytotoxique dans le traitement de la moelle osseuse de patients leucémiques à qui la moelle osseuse ainsi traitée sera ultérieurement transplantée. C'est également le cas lorsque l'imunotoxine est utilisée pour réduire la population de cellules T d'un greffon en vue d'applications de greffe allogénique de moelle osseuse dans le but de prévenir les désordres liés à la réaction du greffon contre l'hôte.

Par contre, lorsque l'immunotoxine est utilisée in vivo en tant qu'agent thérapeutique chez l'homme, l'emploi in vivo d'un ionophore afin de bénéficier de l'effet potentialisateur connaît certaines limitations inhérentes:

- à la toxicité propre des ionophores;
- à leur faible solubilité dans les solvants appropriés aux admistrations parentérales;
- à leur élimination très rapide du plasma sanguin.

Selon la présente invention, il a été trouvé que, d'une façon surprenante, ces limitations pouvaient être éliminées en remplaçant les ionophores carboxyliques par des conjugués associant par liaison covalente un ionophore carboxylique monovalent et une macromolécule, telle qu'un anticorps, un fragment d'anticorps, une protéine, un peptide ou encore une macromolécule mixte obtenue par couplage covalent d'un anticorps et d'une protéine.

Selon un premier aspect, la présente invention a donc pour objet, en tant que produits nouveaux, des conjugués (molécules artificielles mixtes) construits par liaison covalente d'un ionophore carboxylique monovalent avec des macromolécules (tels des anticorps, des protéines, des ligands peptidiques).

Dans la suite de la présente description, de tels conjugués seront désignés sous le nom de "conjugués ionophores".

Les ionophores utilisés sont des molécules connues: ce sont notamment des substances naturelles isolées à partir de souches de champignons Streptomycetes qui comportent un squelette hydrocarboné dans lequel sont inclus des hétérocycles oxygénés. A une extrémité de la chaîne, il existe toujours une fonction acide carboxylique, tandis que, à l'autre extrémité, se trouvent une ou plusieurs fonctions alcool (B.C. Pressman, Annual review of Biochemistry, 45, 501 - 530, 1976).

Ces substances naturelles, ainsi que certains composés hémisynthétiques qui en dérivent, possèdent en commun une activité ionophore, c'est-à-dire la capacité de permettre le transfert d'ions métalliques (et notamment d'ions monovalents) d'une phase hydrophile à une phase lipophile non miscibles.

Les macromolécules utilisées peuvent être des anticorps (ou des fragments d'anticorps), des protéines (telles des hormones peptidiques ou des protéines humaines, par exemple la sérum-albumine humaine), des ligands peptidiques (tels des polypeptides naturels ou synthétiques et, en particulier, la polylysine) ou encore des macromolécules mixtes obtenues par couplage covalent d'un anticorps et d'une protéine.

Dans le cas où l'on utilise un anticorps, celui-ci peut être soit de caractère polyclonal s'il résulte d'une immunisation classique conduite chez l'animal, soit de caractère monoclonal s'il est produit par un clone de cellules hybrides obtenues par fusion entre lymphocytes et cellules de myélome. Cet anticorps pourra être utilisé soit sous la forme de molécules entières d'immunoglobuline comportant la capacité à reconnaître l'antigène choisi, soit sous la forme de tout fragment de ces molécules d'immunoglobuline ayant conservé la capacité à reconnaître l'antigène choisi, et en particulier les fragments connus sous les appellations de F(ab')2, Fab et Fab'.

Le couplage chimique entre les macromolécules et l'ionophore peut être réalisé par diverses méthodes sous réserve que la méthode choisie:

- préserve les activités biologiques respectives des composants du conjugué;
- assure une reproductibilité satisfaisante et un bon rendement de couplage;
- permette de maîtriser la valeur du rapport ionophore/macromolécule dans le conjugué obtenu;
- conduise à un produit stable et soluble dans l'eau.

Parmi toutes les méthodes de couplage chimique répondant à ces caractéristiques, on peut choisir celles qui mettent en oeuvre une ou plusieurs fonctions thiol pour l'établissement de la liaison. Dans ce cas, on peut utiliser un groupement thiol artificiellement introduit sur l'un des composés à coupler, et introduire sur l'autre composé une ou plusieurs fonctions susceptibles de réagir avec les fonctions thiol, et ceci en milieu aqueux de pH compris entre 5 et 9, à une température ne dépassant pas 30° C, pour fournir une liaison stable, covalente et définie.

Par exemple, on peut introduire artificiellement un thiol sur les ionophores par action de l'anhydride S-acétyl-mercapto-succinique qui sera capable d'acyler une des fonctions alcool de l'ionophore. On pourra ensuite libérer cette fonction thiol par élimination du radical acétyle protecteur par action de l'hydroxylamine ainsi que cela a déjà été décrit (Archives of Biochemistry and Biophysics, 119, 41 - 49, 1967).

Des ionophores convenables sont la monensine, la nigéricine, la grisonixine et le lasalocide; la monensine et la nigéricine, ayant les fomules:

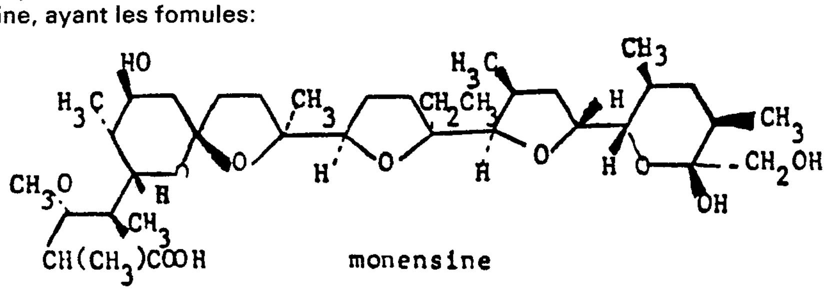

monensine

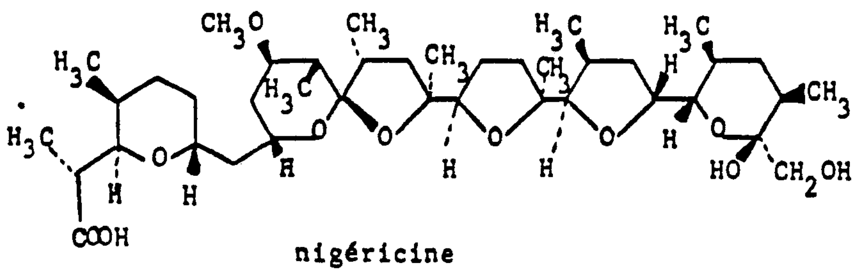

nigéricine

sont particulièrement préférées.

L'introduction du thiol artificiel est effectuée par réaction de l'ionophore avec l'anhydride S-acétyl-mercaptosuccinique de formule

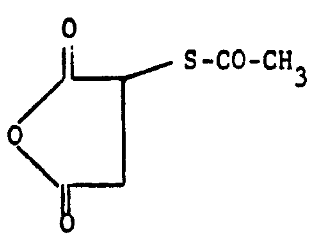

dans un solvant organique inerte à environ la température ambiante.

Par cette réaction, on établit une liaison ester entre un des hydroxyles de l'ionophore, l'hydroxyle primaire dans le cas de la monensine et de la nigéricine, et l'acide acétylmercaptosuccinique et on obtient un composé de formule:

$$I°\text{-O-CO-}\underset{\displaystyle CH_2COOH}{\underset{|}{C}}H\text{-S-CO-}CH_3 \qquad I$$

où I° représente le résidu du ionophore privé d'un de ses hydroxyles, l'hydroxyle primaire dans le cas de la monensine et de la nigéricine.

Le groupe thiol du composé ci-dessus peut être libéré par action de l'hydroxylamine et le produit ainsi obtenu, de formule:

$$I°\text{-O-CO-}\underset{\displaystyle CH_2COOH}{\underset{|}{C}}H\text{-SH} \qquad II$$

où I° est tel que défini ci-dessus, peut être utilisé in situ pour l'opération de couplage.

Lesdits ionophores activés, qui sont représentés par la formule:

$$I°\text{-O-CO-}\underset{\displaystyle CH_2COOH}{\underset{|}{CH}}\text{-S-W} \qquad III$$

dans laquelle W est l'hydrogène ou un groupe acétyle, sont nouveaux et représentent un autre aspect de la présente invention.

Le groupe

$$I°\text{-O-CO-}\underset{\displaystyle CH_2COOH}{\underset{|}{CH}}$$

est indiqué ci-après I et désigne "l'ionophore à coupler".

Le couplage sur la macromolécule peut être effectué avec l'un ou l'autre des composés I et II, mais ledit couplage est plus rapide avec le composé II qui est donc utilisé de préférence.

Le couplage sera alors réalisé avec la macromolécule sur laquelle on aura introduit une ou plusieurs fonctions susceptibles d'établir une liaison covalente avec le thiol. Cette liaison covalente pourra être soit une liaison disulfure, soit une liaison thioéther.

**Cas de la liaison disulfure**

Dans ce cas, la préparation du conjugué peut être représentée par le schéma:

ISH + P-R-S-S-X → I-S-S-R-P + XSH

dans lequel:

I représente l'ionophore à coupler,

P la macromolécule à modifier,

-S-S-X désigne un groupement disulfure mixte activé dont X est le radical activateur.

La macromolécule substituée par un atome de soufre activé est obtenue à partir de la macromolécule elle-même, par substitution à l'aide d'un réactif lui-même porteur d'un atome de soufre activé selon le schéma:

P + Y-R-S-S-X → P-R-S-S-X

dans lequel:

P est la macromolécule à modifier,

Y représente une fonction permettant la fixation covalente du réactif sur la protéine,

R désigne un groupement pouvant porter simultanément les substituants Y et -S-S-X,

X désigne le radical activateur.

Le groupement fonctionnel Y est une fonction capable de se lier de façon covalente avec l'une quelconque des fonctions portées par les chaînes latérales des aminoacides constitutifs de la protéine à substituer. Parmi celles-ci, les fonctions aminées terminales des radicaux lysyle contenus dans la protéine sont particulièrement indiquées. Dans ce cas, Y pourra notamment représenter:

- un groupe carboxylique qui pourra se lier aux fonctions aminées de la protéine en présence d'un agent de couplage tel qu'un carbodiimide et notamment un dérivé soluble dans l'eau comme l'éthyl-1 (diéthylamino-3 propyl)-3-carbodiimide,
- un chlorure d'acide carboxylique qui est susceptible de réagir directement avec les fonctions aminées pour les acyler,
- un ester dit "activé" tel qu'un ester d'ortho- ou para-, nitro- ou dinitro-phényle, ou encore un ester de N-hydroxysuccinimide qui réagit directement avec les fonctions aminées pour les acyler,
- un anhydride interne d'un diacide carboxylique tel, par exemple, l'anhydride succinique qui réagit spontanément avec les fonctions amines pour créer des liaisons amides,

- un groupement imidoester 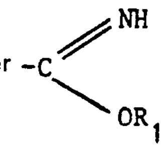

où $R_1$ est un groupe alkyle réagissant avec les groupes aminés de la macromolécule selon la réaction:

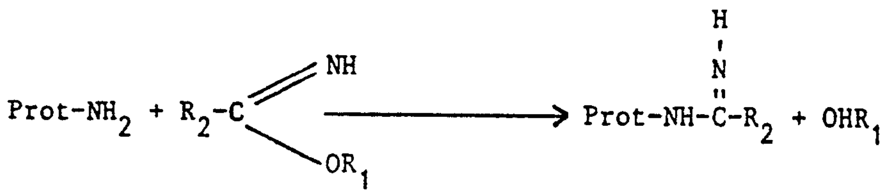

Le radical -S-S-X désigne un disulfure mixte activé capable de réagir avec un radical thiol libre. En particulier, dans le disulfure mixte, X pourra désigner un groupe pyridyl-2 ou pyridyl-4 éventuellement substitué par un ou des radicaux alkyle, halogène, carboxylique. X peut aussi désigner un groupe phényle de préférence substitué par un ou des groupes nitro ou carboxylique. X peut encore représenter un groupe alcoxycarbonyle, tel que le groupe méthoxycarbonyle.

Le radical R désigne tout radical capable de porter simultanément les substituants Y et -S-S-X. Il devra être choisi de façon à ne pas comporter de fonctions susceptibles d'interférer au cours des réactions ultérieures avec les réactifs utilisés et les produits synthétisés. En particulier, le groupe R peut être un groupe $-(CH_2)_n$ avec n compris entre 1 et 10, ou encore un groupe:

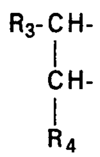

dans lequel $R_4$ désigne l'hydrogène ou un groupe alkyle ayant de 1 à 8 atomes de carbone, et $R_3$ désigne un substituant inerte vis-à-vis des réactifs utilisés ultérieurement, tel qu'un groupe carbamate

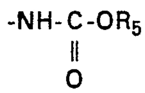

où $R_5$ désigne un groupe alkyle droit ou ramifié ayant de 1 à 5 atomes de carbone et notamment le groupe tertiobutyle.

La réaction du composé Y-R-S-S-X avec la macromolécule P est effectuée en phase liquide homogène, le plus souvent dans l'eau ou une solution tampon. Lorsque la solubilité des réactifs l'exige, il est possible d'ajouter au milieu réactionnel jusqu'à 30 % en volume d'un solvant organique miscible à l'eau, tel qu'un alcool et notamment le butanol tertiaire. La réaction est effectuée à température ambiante pendant un temps variant de quelques minutes à 24 h. Après quoi, une dialyse permet d'éliminer les produits de faible masse moléculaire, et en particulier les excès de réactifs. Ce procédé permet d'introduire un nombre de substituants par mole de macromolécule habituellement compris entre 1 et 50.

En utilisant de tels composés, la couplage de la macromolécule et de l'ionophore est réalisé par mise en présence en solution aqueuse des deux composés à une température ne dépassant pas 30° C pendant un temps variant de quelques minutes à 1 jour. La solution aqueuse obtenue est dialysée pour éliminer les produits de faible masse moléculaire.

**Cas de la liaison thioéther**

La préparation du conjugué consiste alors à faire réagir I-SH avec la protéine P, sur laquelle aura préalablement été introduit un radical maléimide. La réaction est alors représentée par le schéma:

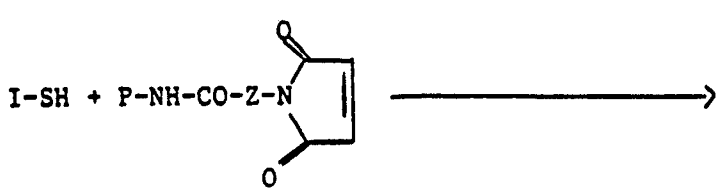

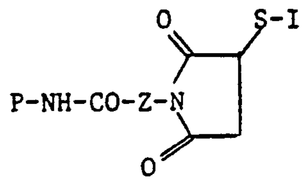

dans lequel:

Z représente une structure d'espacement aliphatique ou aromatique comportant de 1 à 10 atomes de carbone.

La protéine P substituée par le maléimide est obtenue à partir de la protéine P elle-même, par substitution de fonctions aminées de la protéine à l'aide d'un réactif lui-même porteur du groupement maléimide, selon le schéma:

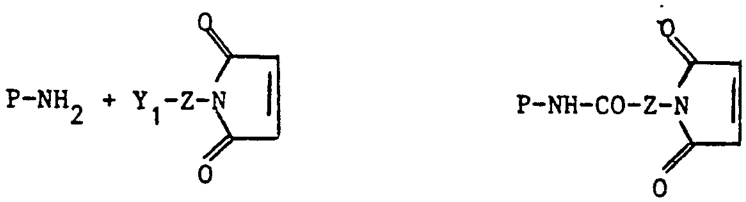

dans lequel $Y_1$ représente:

- soit un groupe carboxylique, la réaction étant alors effectuée après activation de la fonction carboxylique en présence d'un agent de couplage, tel qu'un carbodiimide et notamment un dérivé soluble dans l'eau, tel que l'éthyl-1 (diéthylamino-3 propyl)-3-carbodiimide,
- soit un ester dit "activé" tel qu'un ester d'ortho- ou para-, nitro- ou dinitrophényle, ou encore un ester de N-hydroxysuccinimide qui réagit directement avec les fonctions aminées pour les acyler.

La préparation de tels réactifs est notamment décrite dans Helvetica Chimica Acta, 58, 531 - 541 (1975). D'autres réactifs de la même classe sont commercialement disponibles.

La réaction du composé 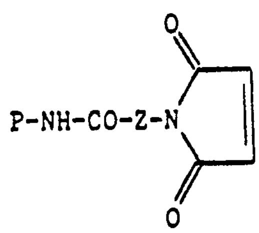 avec l'ionophore

avec l'ionophore est effectuée en phase liquide homogène, le plus souvent dans l'eau ou une solution tampon. Lorsque la solubilité des réactifs l'exige, il est possible d'ajouter au milieu réactionnel jusqu'à 20 % en volume d'un solvant organique miscible à l'eau, tel qu'un alcool et notamment le butanol tertiaire.

La réaction est effectuée à température ambiante pendant un temps variant de quelques heures à 24 heures. Après quoi, une dialyse permet d'éliminer les produits de faible masse moléculaire et, en particulier, les excès de réactifs. Ce procédé permet d'introduire un nombre de groupements substituants par mol de protéine habituellement compris entre 1 et 50.

En utilisant de tels composés, le couplage de la macro-molécule avec l'ionophore est réalisé par mise en présence en solution aqueuse des deux composés à une température ne dépassant pas 30°C pendant un temps variant de quelques heures à un jour. La solution obtenue est dialysée pour éliminer les produits de faible masse moléculaire, puis le conjugué peut être purifié par diverses méthodes connues.

Selon un second aspect, l'invention concerne l'utilisation des conjugués ionophores comme potentialisateurs des immunotoxines. Elle concerne également les associations médicamenteuses contenant au moins une immunotoxine et au moins un ionophore selon l'invention.

Les études effectuées sur les conjugués ionophores ont montré que:

- l'effet potentialisateur des ionophores est préservé après couplage avec la macromolécule.

La liaison de l'ionophore à la macromolécule apporte à celui-ci une grande solubilité en milieu aqueux favorable à l'administration in vivo du produit.

- la liaison de l'ionophore à la macromolécule allonge considérablement le temps de demi-vie plasmatique de

l'ionophore, ce qui est essentiel au maintien de l'effet potentialisateur in vivo;
- la toxicité du conjugué est plus faible que celle de l'ionophore lui-même.

Ainsi, pour chaque patient, on dirigera vers les cellules-cibles au moins deux substances effectrices indispensables au résultat:

- d'une part, une protéine cytotoxique (telle que par exemple la chaîne A de ricine) qui sera l'effecteur cytotoxique inclus dans le conjugué dit immunotoxine;
- d'autre part, l'ionophore (tel que par exemple la monensine) qui est le constituant du système potentialisateur inclus dans le conjugué dit "conjugué ionophore".

Dans le cas où l'ionophore est couplé à une macromolécule possédant un récepteur à la surface des cellules-cibles (par exemple lorsque la macromolécule sera un anticorps ou un fragment d'anticorps reconnaissant un antigène spécifique de la population cellulaire à détruire différent de celui reconnu par l'immunotoxine) ou bien si l'ionophore est couplé à une hormone peptidique possédant un récepteur à la surface des cellules à détruire, la probabilité pour que les deux cibles choisies soient présentes ensemble à la surface des cellules non cibles devient très faible et l'on dispose ainsi d'un moyen extrêmement puissant pour augmenter encore le caractère spécifique de la cytotoxicité des immunotoxines.
Les exemples suivants illustrent l'invention sans en limiter la portée.

**Exemple 1**

Conjugué ionophore obtenu par réaction entre l'anticorps T 29 - 33 substitué par un groupement disulfure activé et la monensine sur laquelle on a introduit un thiol.

a) Anticorps T 29 - 33

Cet anticorps est un anticorps monoclonal dirigé contre l'antigène T 200 des leucocytes humains. Cet anticorps est décrit dans J. Exp. Med., 1980, 152, 842. Il est commercialement disponible auprès d'Hybritech Inc., San Diego - California - USA.

b) Anticorps T 29 - 33 activé

A 2 ml d'une solution d'anticorps T 29 - 33 à 10 mg/ml (soit 0,133 µmol d'anticorps) est ajoutée une solution aqueuse contenant 3 mg d'acide (pyridyl-2-disulfanyl)-3-propionique préalablement dissous dans le butanol tertiaire et 1,8 mg d'éthyl-1-diméthylamino-3-propyl-3-carbodiimide. Le mélange est agité 15 min à 30°C puis dialysé en continu contre du tampon phosphate 125 mM pH 7 (40 h à 500 ml/h). Après dialyse, la solution protéique est centrifugée et l'on obtient 2,5 ml d'une solution à 6,6 mg d'anticorps modifié par ml. Par dosage spectrophotométrique à 343 nm de la pyridine thione-2 libérée par échange avec le mercapto-2-éthanol, on constate que l'on a obtenu un anticorps portant 7,2 groupements activateurs par mol d'anticorps.

c) Monensine activée

La monensine utilisée est un produit commercial. Elle a été modifiée comme suit: 693 mg de monensine sont dissous dans le chloroforme puis mis en réaction avec 350 mg d'anhydride S-acétylmercaptosuccinique (SAMSA). On laisse la réaction évoluer une 1/2 h à température ambiante. Le milieu réactionnel est ensuite évaporé à sec sous vide puis repris dans l'acétate d'éthyle et lavé extensivement à l'eau. La phase organique est ensuite séchée et tirée à sec sous vide. Le produit est obtenu cristallisé après séchage sous vide. Il est identifié par son spectre RMN et son spectre de masse.
Le produit ainsi obtenu (monensine activée S-acétyl) a la formule III ci-dessus, dans laquelle I° est le résidu de la monensine privée de son hydroxyle primaire et W est un groupe acétyle.
Le radical acétyle peut être libéré par action de l'hydroxylamine à une concentration de 50 mM finale et le produit ainsi obtenu, répondant à la formule III ci-dessus où I° est le résidu de monensine privée de son hydroxyle primaire et W est l'hydrogène, peut être directement utilisé pour le couplage.
Toutefois, pour l'étape suivante il est préférable de libérer le groupe thiol in situ en présence de la monensine activée S-acétyl et de l'anticorps activé.

d) Préparation du conjugué ionophore

8 mg (soit 9 µmol) de monensine activée S-acétyl sont dissous dans le minimum de terbutanol et ajoutés à 2,5 ml de la solution d'anticorps activé à 6,6 mg/ml (soit 0,11 µmol) dans le tampon phosphate 125 mM pH 7. On ajoute 125 µl d'une solution d'hydroxylamine 1N.
L'incubation dure 2 h à 25°C puis le milieu réactionnel est purifié par dialyse de l'excès de réactifs contre du

tampon PBS (phosphate 10 mM, chlorure de sodium 140 mM pH 7,4).

Après dialyse et centrifugation, on a obtenu 2,8 ml d'une solution d'IgG T 29 - 33 à 5,6 mg/ml portant en moyenne 7 monensines par mol d'anticorps.

**Exemple 2**

Potentialisation de l'immunotoxine anti-T65

Le conjugué selon l'invention, obtenu comme indiqué précédemment, a été étudié en ce qui concerne ses propriétés biologiques et plus spécialement sa capacité à potentialiser l'activité de l'immunotoxine anti-T65 dans un modèle cellulaire approprié.

Ce modèle est constitué par des cellules de la lignée lymphoblastoïde humaine CEM qui portent naturellement les antigènes T65 et T200. L'antigène T65 contre lequel est dirigée l'immunotoxine utilisée constitue le premier antigène-cible du modèle. Cette immunotoxine est celle qui a été décrite dans une précédente demande de brevet de la demanderesse déposée en France sous le n° 81/21836. L'antigène T200 contre lequel est dirigé le conjugué ionophore sera le deuxième antigène-cible du modèle.

La propriété fondamentale des immunotoxines étant d'inhiber la synthèse protéique des cellules-cibles, le test utilisé consiste à mesurer l'effet des substances étudiées sur l'incorporation de 14C-leucine dans les cellules cancéreuses en culture. Cette mesure est effectuée selon une technique adaptée de celle décrite dans Journal of Biological Chemistry, 1974, 249 (11), 3557 - 3562, utilisant le traceur 14C-leucine pour la détermination du taux de synthèse protéique. La détermination de la radioactivité incorporée est effectuée ici sur les cellules entières isolées par filtration.

A partir de ces déterminations, on peut tracer les courbes effets/doses présentant en abscisses la concentration molaire en chaîne A des substances étudiées et en ordonnées l'incorporation de 14C-leucine exprimée en pourcentage de l'incorporation des cellules témoins en l'absence de toute substance affectant la synthèse protéique.

On peut ainsi déterminer pour chaque substance étudiée la concentration qui inhibe 50 % de l'incorporation de 14C-leucine ou "concentration inhibitrice 50" (CI 50).

Les différents essais ont été conduits comme suit; les résultats expérimentaux correspondants sont présentés sur la figure 1.

a) Des cellules CEM sont incubées 18 h en présence de concentrations connues d'immunotoxine anti-T65 utilisée à titre de référence, puis les cellules sont soumises à l'étape d'incorporation du traceur radioactif. La CI 50 obtenue est de $2{,}8 \cdot 10^{-11}$ M (courbe 1), ce qui montre que les cellules sont normalement sensibles à l'effet de l'immunotoxine.

b) Les cellules CEM sont incubées 18 h à 37°C en présence d'un mélange de concentrations connues d'immunotoxine anti-T65 et respectivement:

1 - soit de monensine libre à la concentration de 50 nM (courbe 2);

2 - soit de conjugué ionophore anti-T200 préalablement décrit, à la concentration de $10^{-8}$ M (soit 70 nM en monensine) (courbe 3).

Il a été préalablement vérifié que la monensine et le conjugué ionophore ne sont pas cytotoxiques pour les cellules employées aux concentrations indiquées.

Les CI 50 obtenues sont respectivement:

$2{,}4 \cdot 10^{-14}$ M pour la monensine 50 nM

$1{,}4 \cdot 10^{-14}$ M pour le conjugué ionophore $10^{-8}$ M

Ces résultats montrent des effets potentialisateurs remarquables, d'un facteur de 1000 fois pour la monensine 50 nM et de 2000 fois pour le conjugué ionophore à la concentration de $10^{-8}$ M (soit 70 nM en monensime).

**Exemple 3**

Conjugué ionophore obtenu par réaction entre l'anticorps 3E10 substitué par un groupement disulfure activé et la monensine sur laquelle on a introduit un thiol.

a) Anticorps 3E10

Cet anticorps est un anticorps monoclonel dirigé contre un antigène membranaire humain. Il a été obtenu lors d'une fusion lymphocytaire anti-cellules de mélanome humaines SK MEL 28 "WOODBURY R.G. ET AL.

PROCEEDING NATIONAL ACADEMY OF SCIENCES 77 p. 2183 - 2186 (1980). L'hybridome a été cloné et multiplié; l'anticorps a été produit en ascite puis purifié sur protéine A Sepharose®.

b) Anticorps 3E10 activé

Cet anticorps est obtenu à partir de l'anticorps ci-dessus selon une technique décritc à l'exemple 1. On a ainsi obtenu 195 mg d'anticorps 3E10 possédant 8 groupements activateurs par mol d'anticorps.

c) Monensine activée

La monensine est activée selon la méthode décrite à l'exemple 1.

d) Préparation du conjugué ionophore

91 mg de monensine activée S-acétyl (soit 104 µmol) sont dissous dans le minimum de terbutanol et ajoutés à 50 ml de la solution d'anticorps activé à 3,9 mg/ml (soit 1,3 µmol) dans le tampon phosphate 125 mM pH 7. On ajoute 2,5 ml d'une solution d'hydroxylamine 1 N.

L'incubation dure 2 h à 25°C, puis le milieu réactionnel est purifié de l'excès de réactifs par dialyse contre du tampon PBS (phosphate 10 mM, chlorure de sodium 140 mM, pH 7,4). Après dialyse et centrifugation, on a obtenu 2,8 ml d'une solution d'IgG à 3,7 mg/ml portant en moyenne 8 monensines par IgG.

**Exemple 4**

Toxicité du conjugué 3E10-monensine

Le conjugué 3E10-monensine a été étudié en ce qui concerne ses propriétés biologiques in vivo. Plus spécialement, sa toxicité et sa pharmacocinétique ont été comparées à celles de la monensine libre.

La toxicité de la monensine libre a été déterminée chez la souris. La dose létale 50 % après injection I.V. est de 4 mg/kg, soit 80 µg/souris.

La toxicité du conjugué a été testée sur souris en administration unique également par voie I.V. Pour les souris de chaque lot, le produit a été injecté par voie intraveineuse aux doses suivantes 3,7 mg, 1,85 mg, 0,952 mg, 0,496 mg de conjugé, soit respectivement l'équivalent de 163 µg, 81,5 µg, 40,75 µg, 20,4 µg de monensine. Aucune mortalité n'a été observée dans aucun des lots de souris.

**Exemple 5**

Pharmacocinétiquc du conjugué 3E10-monensine.

La pharmacocinétique a été étudiée chez la souris nude femelle à l'aide du conjugué ionophore décrit à l'exemple 3.

On injecte aux souris:

- soit 1 ml de conjugé ionophore à 3,7 mg/ml (soit 163 µg de monensine couplée),
- soit 163 µg de monensine libre (lot témoin).

Pour chaque temps, les plasmas de deux souris sont prélevés. La monensine active est dosée dans les plasmas par dilution de ceux-ci et comparaison avec une courbe-étalon de monensine libre dans le test d'inhibition de la synthèse protéique décrit à l'exemple 1.

Le dosage est effectué sur des cellules CEM en présence de l'immunotoxine anti-T65.

Les résultats sont présentés à la figure 2 sur laquelle on a porté en abscisses le temps en heures et en ordonnées la concentration en monensine en µg/ml. Ces résultats montrent:

1) que la monensine libre ne peut pas être mise en évidence dans les plasmas (sauf au temps zéro où l'on retrouve une concentration plasmatique de $5 \cdot 10^{-7}$ M soit environ 0,5 µg/ml, soit dans la masse sanguine totale de la souris une quantité égale à 0,007 % de la dose injectée).
2) Dans le cas du conjugué, on observe que le conjugué peut être mis en évidence pendant au moins 8 h à une concentration de l'ordre de 10 µg/ml, soit environ $1 \cdot 10^{-5}$ M.

Au bout de 24 h, la concentration retrouvée n'est plus que voisine de 1,5 µg/ml. Cette concentration est cependant 100 fois supérieure à celle nécessaire à l'activation maximale dans les conditions des essais in vitro.

**Exemple 6**

Conjugué ionophore obtenu par réaction entre la sérum-albumine humaine (SA) substituée par un groupement disulfure activé et la monensine sur laquelle on a introduit un thiol.

a) Sérum-albumine humaine activée

La SA a été obtenue à partir de la SA ci-dessus selon une technique analogue à celle décrite pour les anticorps dans les exemples précédents. On a ainsi obtenu 220 mg de SA possédant 16 groupements activateurs par mol d'albumine.

b) Monensine activée

La monensine est activée selon la méthode décrite à l'exemple 1.

c) Préparation du conjugué ionophore

228 mg de monensine activée S-acétyl, soit 268 µmol, sont dissous dans le minimum de terbutanol et ajoutés à 23 ml de la solution de SA activée à 9,4 mg/ml (soit 52,7 µmol) dans le tampon phosphate 125 mM pH 7. On ajoute 1,15 ml d'une solution d'hydroxylamine 1 N.

L'incubation dure 1 h à 25°C, puis le milieu réactionnel est dialysé contre du tampon PBS (phosphate 10 mM, chlorure de sodium 140 mM, pH 7,4).

Après dialyse et centrifugation, on a obtenu 28 ml d'une solution de SA modifiée à 8,7 mg/ml portant en moyenne 16 monensines par mol d'albumine.

**Exemple 7**

Pharmacocinétique du conjugué SA-monensine

La pharmacocinétique a été étudiée chez la souris nude mâle à l'aide du conjugué ionophore décrit à l'exemple 6.

Le protocole expérimental utilisé est le même que celui décrit à l'exemple 5.

Les résultats sont présentés à la figure 3 sur laquelle on a porté en abscisses le temps en h et en ordonnées la concentration en monensine en µg/ml. Ils montrent que:

1) Comme dans le cas de l'exemple 5, la monensine libre ne peut pas être mise en évidence dans les plasmas.
2) On observe que le conjugué peut être mis en évidence pendant au moins 4 h à une concentration de l'ordre de 30 µg/ml. Au bout de 8 h, la concentration retrouvée n'est plus que de l'ordre de 6 µg/ml. Cette concentration est cependant 400 fois supérieure à celle nécessaire à l'activation maximale dans les conditions des essais in vitro.

**Exemple 8**

Conjugué ionophore obtenu par réaction entre le fragment F(ab')2 de l'anticorps anti-T65 substitué par un groupement disulfure activé et la monensine sur laquelle on a introduit un thiol.

a) Fragment F(ab')2 anti-T65

Le fragment F(ab')2 anti-T65 a été obtenu à partir de l'anticorps anti-T65 précédemment décrit par hydrolyse enzymatique à l'aide de pepsine. L'anticorps anti-T65 est préalablement dialysé contre du tampon formiate de sodium 0,1 M pH 3,7, puis 176 mg d'anticorps sont mis à incuber en présence de pepsine (0,05 mg de pepsine par mg d'anticorps) pendant 2 heures à 37°C. La réaction enzymatique est ensuite arrêtée par 2,05 ml de tampon tris 2M.

La solution est centrifugée puis purifiée par filtration sur une colonne ACA 44 avec mesure de la densité optique de l'effluent à 280 nm. Par regroupement des fractions purifiées et après concentration, on obtient 26 ml d'une solution à 3,3 mg/ml.

b) Fragment F(ab')2 anti-T65 activé

Le produit a été obtenu à partir du fragment F(ab')2 décrit ci-dessus selon une technique analogue à celle

décrite pour les anticorps dans l'exemple 1-b. On a obtenu 0,5 mg de F(ab')2 anti-T65 possédant 34,3 groupements activateurs par mol d'anticorps.

c) Monensine activée

La monensine est activée selon la méthode décrite à l'exemple 1-c.

d) Préparation du conjugué ionophore

0,44 mg (soit 0,52 µmol) de monensine sont dissous dans le minimum de terbutanol et ajoutés à 1,02 ml de la solution de F(ab')2 activé à 0,33 mg/ml (soit 0,003 µmol) dans le tampon phosphate 125 mM pH 7 en présence de 54 µl d'une solution d'hydroxylamine 1M.

L'incubation dure 1 heure à 25° C puis le milieu réactionnel est purifié par dialyse de l'excès de réactifs contre du tampon PBS (phosphate 10 mM, chlorure de sodium 140 mM pH 7,4). Après dialyse et centrifugation, on a obtenu 1,1 ml d'une solution de F(ab')2 anti-T65 à 0,33 mg/ml portant en moyenne 34 monensines par F(ab')2.

**Exemple 9**

Conjugué ionophore obtenu par réaction entre la sérum-albumine humaine substituée par des groupements disulfure activés et d'une part, l'anticorps anti-DNP sur lequel on a introduit un thiol et, d'autre part, la monensine sur laquelle on a introduit un thiol. On désigne par anticorps anti-DNP des anticorps spécifiquement dirigés contre le radical dinitro-2,4 phényle.

a) Préparation de l'anticorps anti-DNP

Les anticorps anti-DNP utilisés dans cet exemple sont obtenus selon le mode opératoire décrit dans l'exemple 4 du brevet FR-78-27838.

30 mg d'une solution d'IgG anti-DNP à 6,5 mg/ml (soit 0,2 µmol) sont rajoutés 40 µl d'une solution d'anhydride S-acétylmercaptosuccinique (SAMSA) à 17 mg/ml dans le DMF. Le milieu réactionnel est agité pendant 2 heures puis purifié de l'excès de réactifs par dialyse contre du tampon phosphate 125 mM pH 7, pendant 24 heures, à un débit de 400 ml/h.

On obtient ainsi 4,5 ml d'une solution à 6,4 mg/ml. Par dosage spectrophotométrique des SH libérés par action de l'hydroxylamine, on constate que l'on a obtenu une IgG portant 4,4 SH par mole d'anticorps.

b) Préparation de la sérum-albumine activée

La sérum-albumine activée a été obtenue à partir de la sérum-albumine humaine selon une technique analogue à celle décrite pour les anticorps dans les exemples précédents. On a ainsi obtenu 30 mg de sérum-albumine possédant 38 groupements activateurs par mol d'albumine.

c) Monensine activée

La monensine est activée selon la méthode décrite à l'exemple 1-c.

d) Préparation du conjugué ionophore

A 4,1 ml de solution de sérum-albumine activée précédemment obtenue (soit 0,450 µmol), on ajoute 3,5 ml de la solution d'anticorps anti-DNP modifié (soit 0,149 µmol). On ajoute 380 µl d'une solution d'hydroxylamine 1 N puis on laisse le mélange évoluer 5 heures à 30° C. Le milieu réactionnel est purifié par filtration sur une colonne de SEPHADEX G200® avec mesure de la densité optique à 280 nm. Le regroupement des fractions contenant à la fois l'anticorps et la sérum-albumine conduit à l'obtention de 16 ml de conjugué qui est purifié de l'excès d'anticorps libres par passage sur une colonne de DEAE trisacryl (IBF). L'élution est réalisée dans du tampon tris 0,02 M pH 8,8 et a l'aide d'un gradient de NaCl de 0'035 M à 0,1 M, avec mesure de la densite optique à 280 nm.

Les fractions contenant à la fois l'anticorps et la sérum-albumine (SAH) sont regroupées, dialysées contre du tampon phosphate 125 mM pH 7, puis concentrées par ultrafiltration. On obtient 12 ml d'une solution de conjugué IgG-SAH. Le taux de couplage moyen pour cette préparation, déterminé par densitométrie d'électrophorèse, est de 1,5 mol de sérum-albumine par mol d'anticorps.

A cette solution sont ajoutés 4,86 mg de monensine activée (soit 5,76 µmol) dissous dans le minimum de terbutanol. On ajoute 600 µl d'une solution d'hydroxylamine 1 N et on laisse le mélange évoluer 1 heure à 25° C puis le milieu réactionnel est dialysé contre du tampon PBS (phosphate 10 mM + chlorure de sodium 140 mM pH 7,4).

Après dialyse et centrifugation, on a obtenu 13,5 ml d'une solution de conjugué IgG-SAH à 0,915 mg/ml portant en moyenne 26 monensines par IgG (soit 17,4 monensines par SAH).

**Exemple 10**

Potentialisation de l'immunotoxine T65

Le conjugué selon l'invention, obtenu comme indiqué à l'exemple 9, a été étudié en ce qui concerne sa capacité à potentialiser l'activité de l'immunotoxine anti-T65 sur le modèle cellulaire décrit à l'exemple 2.

Dans un premier temps, les cellules CEM sont ou non artificiellement marquées par le TNP, c'est-à-dire le groupement trinitro-2,4,6 phényle, molécule cible du conjugué ionophore.

Dans un second temps, ces cellules sont incubées avec le conjugué ionophore et/ou l'immunotoxine anti-T65, puis on procède à la mesure du taux d'inhibition de la synthèse protéique selon la méthode décrite à l'exemple 2.

a) Marquage des cellules par le TNP

Le marquage par le TNP est réalisé à +4°C par incubation de $2 \cdot 10^6$ cellules CEM par ml de tampon PBS avec un égal volume d'une solution à 10 mg/ml de trinitrobenzène-sulfonate de sodium. La réaction est arrêtée après 15 secondes par addition d'un excès d'une solution à $10^{-3}$ M de L-lysine; les cellules sont ensuite lavées.

b) Mesure du taux d'inhibition de la synthèse protéique

Les cellules CEM marquées ou non par le TNP et à la concentration de $5 \cdot 10^5$ par ml sont préincubées 1 heure à 4°C avec le conjugué ionophore ajouté à la concentration de $10^{-7}$ M en anticorps. Après lavages à 4°C, les cellules sont resuspendues dans du milieu RPMI-1640 sans leucine et contenant 10 % de sérum foetal de veau thermo inactivé et des antibiotiques, puis les cellules sont incubées 20 heures à 37°C en présence de concentrations connues d'immunotoxine anti-T65. Les cellules sont ensuite soumises à l'étape d'incorporation du traceur radioactif.

c) Activité de la monensine après couplage (figure 4)

Les cellules CEM non marquées sont directement incubées avec l'immunotoxine à des concentrations variables et avec la monensine couplée ou non, introduite à la concentration fixe de $5 \cdot 10^{-8}$ M. La potentialisation de la cytotoxicité de l'immunotoxine est identique dans les deux cas, ceci démontrant que le couplage de la monensine n'affecte pas ses propriétés activatrices.

d) Spécificité de l'activation de l'imunotoxine (IT) par le conjugué ionophore (figure 5)

- La courbe 1 (contrôle) montre que, après marquage des cellules par le TNP, la préincubation avec l'anticorps anti-DNP à $10^{-7}$ M, puis incubation avec l'IT et la monensine à $5 \cdot 10^{-8}$ M, la potentialisation de l'IT par l'ionophore est pleinement conservée.
- La courbe 2 représente la cytotoxicité obtenue par préincubation des cellules marquées au TNP avec le conjugué ionophore introduit à la concentration de $10^{-7}$ M en anticorps, soit $2{,}6 \cdot 10^{-6}$ M en monensine, suivie de l'incubation avec l'immunotoxine. La CI50 obtenue n'est pas notablement différente de la CI50 obtenue avec la monensine libre (courbe 1).
- La courbe 3 représente l'activité du même conjugué ionophore préincubé avec les mêmes cellules mais non marquées au TNP.

La comparaison des courbes 2 et 3 montre que l'activation obtenue avec le conjugué ionophore est bien spécifique des cellules portant l'antigène (ici l'haptène TNP) correspondant à l'anticorps du conjugué ionophore. Le facteur de spécificité est ici supérieur à 100.

- La courbe 4 montre les résultats obtenus quand les cellules marquées au TNP sont préincubées avec l'anticorps anti-DNP et avec la monensine non couplée. La cytotoxicité de l'immunotoxine est alors très faible et identique à celle obtenue dans les mêmes conditions expérimentales soit sur cellules CEM non marquées au TNP (courbe 5), soit sur cellules CEM marquées au TNP et préincubées avec l'anticorps anti-DNP (courbe 6). Ces résultats de contrôle confirment qu'il est bien nécessaire que la monensine soit couplée à l'anticorps anti-DNP pour que l'activation sélective apparaisse.

Ces résultats démontrent la réalité d'une action cytotoxique, induite par un double ciblage vers les cellules à détruire, à l'aide de deux anticorps différents, d'une part, de la sous-unité toxique constitutive de l'immunotoxine, d'autre part, de l'activateur utilisé pour accroître le potentiel cytotoxique de l'immunotoxine.

**Revendications** pour les Etats Contractants: BE, CH, DE, FR, GB, IT, LI, LU, NL, SE

1. A titre de produits nouveaux, les conjugués obtenus par couplage par liaison covalente d'un ionophore carboxylique monovalent et d'une macromolécule choisie parmi les anticorps, les fragments d'anticorps, les protéines telles que les hormones peptidiques ou les protéines humaines, les ligands peptidiques et les macromolécules mixtes obtenues par couplage covalent d'un anticorps et d'une protéine.

2. Conjugué selon la revendication 1, caractérisé en ce que la macromolécule est un anticorps monoclonal.

3. Conjugué selon l'une des revendications 1 ou 2, caractérisé en ce que le couplage par liaison covalente est obtenu par une liaison disulfure ou une liaison thioéther.

4. Conjugué selon l'une quelconque des revendications 1 à 3, caractérisé en ce que le ionophore possède un groupe hydroxyle primaire.

5. Conjugué selon l'une quelconque des revendications 1 à 4, caractérisé en ce que l'ionophore est la monensine, la nigéricine, la grisonixine et le lasalocide.

6. Conjugué selon l'une quelconque des revendications 1 à 5, caractérisé en ce que la macromolécule est choisie parmi l'anticorps T 29-33, l'anticorps 3E10, l'anticorps anti-DNP et la sérum-albumine humaine.

7. Procédé pour l'obtention des conjugués selon l'une quelconque des revcndications 1 à 6, caractérisé en ce qu'il consiste:

1) à introduire un groupement thiol sur l'un des composés à coupler;

2) à introduire sur l'autre composé une ou plusieurs fonctions susceptibles de réagir avec lesdites fonctions thiol;

3) à faire réagir les deux composés ainsi obtenus en milieu aqueux, à pH compris entre 5 et 9 à une température inférieure à 30° C.

8. Procédé selon la revendication 7, caractérisé en ce que l'on fait réagir l'ionophore portant un groupement thiol avec une macromolécule sur laquelle on a introduit un groupement susceptible de réagir avec la fonction thiol, ce groupement étant de formule -S-S-X où X représente un radical activateur.

9. Procédé selon la revendication 7, caractérisé en ce que l'on fait réagir l'ionophore portant un groupement thiol avec une macromolécule sur laquelle on a introduit un groupement susceptible de réagir avec la fonction thiol, ce groupement étant de formule:

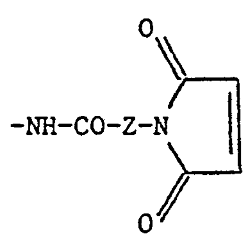

dans laquelle Z est une structure d'espacement aliphatique ou aromatique comportant de 1 à 10 atomes de carbone.

10. Procédé selon la revendication 9, caractérisé en ce que l'introduction du thiol sur l'ionophore est effectuée par réaction de l'ionophore avec l'anhydride S-acétyl-mercaptosuccinique et fournit un composé de formule:

I°-O-CO-CH-S-CO-$CH_3$ I
|
$CH_2COOH$

dans laquelle I° représente le résidu du ionophore privé d'un de ses hydroxyles et en ce que le groupe thiol du composé ci-dessus est libéré par action de l'hydroxylamine pour fournir le composé de formule:

I°-O-CO-CH-SH
|
$CH_2COOH$

dans laquelle I° est tel que défini ci-dessus.

11. Procédé selon la revendication 10, caractérisé en ce que I° est la monensine ou la nigéricine privées de leur hydroxyle primaire.

12. Application des conjugués selon l'une quelconque des revendications 1 à 6, à titre d'agents potentialisateurs des immunotoxines.

13. Association médicamenteuse, caractérisée en ce qu'elle comporte au moins une immunotoxine et au moins un conjugué selon l'une quelconque des revendications 1 à 6.

14. Ionophores carboxyliques activés contenant un groupe thiol, éventuellement acétylé, introduit artificiellement par action de l'anhydride S-acétylmercaptosuccinique et élimination éventuelle du groupe acétyle par l'hydroxylamine.

15. Ionophores carboxyliques activés selon la revendication 14 répondant à la formule:

I°-O-CO-CH-S-W
|
$CH_2COOH$

dans laquelle I° représente le résidu d'un ionophore privé d'un de ses hydroxyles et W représente l'hydrogène ou un groupe acétyle.

16. Ionophores carboxyliques activés selon la revendication 15, ayant la formule:

I°-O-CO-CH-S-W
|
$CH_2COOH$

dans laquelle I° est la monensine ou la nigéricine privées de leur hydroxyle primaire.

**Revendications** pour l'Etat Contractant: AT

1. Procédé pour l'obtention d'un conjugué entre un ionophore carboxylique monovalent et une macromolécule, caractérisé en ce qu'il consiste à coupler par liaison covalente ledit ionophore carboxylique monovalent avec une macromolécule choisie parmi les anticorps, les fragments d'anticorps, les protéines telles que les hormones peptidiques ou les protéines humaines, les ligands peptidiques et les macromolécules mixtes obtenues par couplage covalent d'un anticorps et d'une protéine.

2. Procédé selon la revendication 1, caractérisé en ce que la macromolécule est un anticorps monoclonal.

3. Procédé selon l'une des revendications 1 ou 2, caractérisé en ce que le couplage par liaison covalente est réalisé par une liaison disulfure ou une liaison thioéther.

4. Procédé selon l'une quelconque des revendications 1 à 3, caractérisé en ce que le ionophore possède un groupe hydroxyle primaire.

5. Procédé selon l'une quelconque des revendications 1 à 4, caractérisé en ce que l'ionophore est la monensine, la nigéricine, la grisonixine et le lasalocide.

6. Procédé selon l'une quelconque des revendications 1 à 5, caractérisé en ce que la macromolécule est choisie parmi l'anticorps T29-33, l'anticorps 3E10, l'anticorps anti-DNP ou la sérum-albumine humaine.

7. Procédé selon l'une quelconque des revendications 1 à 6, caractérisé en ce qu'il consiste:

1) à introduire un groupement thiol sur l'un des composés à coupler;

2) à introduire sur l'autre composé une ou plusieurs fonctions susceptibles de réagir avec lesdites fonctions thiol;

3) à faire réagir les deux composés ainsi obtenus en milieu aqueux, à pH compris entre 5 et 9 à une température inférieure à 30° C.

8. Procédé selon la revendication 7, caractérisé en ce que l'on fait réagir l'ionophore portant un groupement thiol avec une macromolécule sur laquelle on a introduit un groupement susceptible de réagir avec la fonction thiol, ce groupement étant de formule -S-S-X où X représente un radical activateur.

9. Procédé selon la revendication 7, caractérisé en ce que l'on fait réagir l'ionophore portant un groupement thiol avec une macromolécule sur laquelle on a introduit un groupement susceptible de réagir avec la fonction thiol, ce groupement étant de formule:

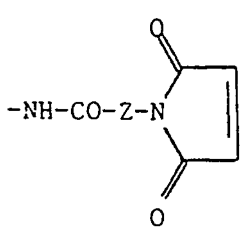

dans laquel le Z est une structure d'espacement aliphatique ou aromatique comportant de 1 à 10 atomes de carbone.

10. Procédé selon la revendication 9, caractérisé en ce que l'introduction du thiol sur l'ionophore est effectuée par réaction de l'ionophore avec l'anhydride S-acétyl-mercaptosuccinique et fournir un composé de formule:

I°-O-CO-CH-S-CO-$CH_3$
|
$CH_2COOH$

dans laquelle I° représente le résidu du ionophore privé d'un de ses hydroxyles et en ce que le groupe thiol du composé ci-dessus est libéré par action de l'hydroxylamine pour fournir le composé de formule:

I°-O-CO-CH-SH
|
$CH_2COOH$

dans laquelle I° est tel que défini ci-dessus.

11. Procédé selon la revendication 10, caractérisé en ce que I° est la monensine ou la nigéricine, privées de leur hydroxyle primaire.

**Patentansprüche** für die Vertragsstaaten: BE, CH, DE, FR, GB, IT, LI, LU, NL, SE

1. Als neue Produkte die Konjugate, die durch Kopplung mittels kovalenter Bindung eines monovalenten Carboxylionophors und eines Makromoleküls, ausgewählt aus Antikörpern, Antikörperfragmenten, Proteinen, wie Peptidhormonen oder Humanproteinen, Peptidliganden und durch kovalente Kopplung eines Antikörpers und eines Proteins erhaltenen gemischten Makromolekülen, erhalten sind.

2. Konjugat nach Anspruch 1, dadurch gekennzeichnet, daß das Makromolekül ein monoklonaler Antikörper ist.

3. Konjugat nach einem der Ansprüche 1 oder 2, dadurch gekennzeichnet, daß die Kopplung mittels kovalenter Bindung durch eine Disulfidbindung oder eine Thioetherbindung erzielt ist.

4. Konjugat nach einem der Ansprüche 1 bis 3, dadurch gekennzeichnet, daß das Ionophor eine primäre Hydroxylgruppe besitzt.

5. Konjugat nach einem der Ansprüche 1 bis 4, dadurch gekennzeichnet, daß das Ionophor Monensin, Nigerizin, Grisonixin und Lasalozid ist.

6. Konjugat nach einem der Ansprüche 1 bis 5, dadurch gekennzeichnet, daß das Makromolekül ausgewählt ist aus dem Antikörper T 29-33, dem Antikörper 3E10, dem Antikörper anti-DNP und menschlichem Serumalbumin.

7. Verfahren zur Herstellung der Konjugate nach einem der Ansprüche 1 bis 6, dadurch gekennzeichnet, daß es darin besteht, daß:

1) eine Thiolgruppe in eine der zu koppelnden Verbindungen eingeführt wird;

2) eine oder mehrere mit den Thiolfunktionen reagierfähige Funktionen in die andere Verbindung eingeführt werden;

3) die beiden so erhaltenen Verbindungen in wässerigem Milieu bei einem pH zwischen 5 und 9 und einer Temperatur von unter 30° C reagieren gelassen werden.

8. Verfahren nach Anspruch 7, dadurch gekennzeichnet, daß man das eine Thiolgruppe tragende Ionophor mit einem Makromolekül reagieren läßt, in welches man eine mit der Thiolfunktion reagierfähige Gruppe eingeführt hat, welche Gruppe die Formel -S-S-X aufweist, worin X eine Aktivatorgruppe darstellt.

9. Verfahren nach Anspruch 7, dadurch gekennzeichnet, daß man das eine Thiolgruppe tragende Ionophor mit einem Makromolekül reagieren läßt, in welches man eine mit der Thiolfunktion reagierfähige Gruppe eingeführt hat, welche Gruppe die Formel:

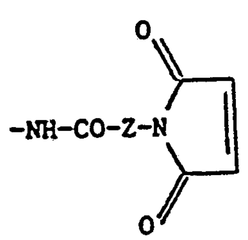

aufweist, worin Z eine aliphatische oder aromatische Raumstruktur mit 1 bis 10 Kohlenstoffatomen darstellt:

10. Verfahren nach Anspruch 9, dadurch gekennzeichnet, daß die Einführung der Thiolgruppe in das Ionophor durch Umsetzung des Ionophors mit dem S-Acetyl-mercaptobernsteinsäureanhydrid erfolgt und eine Verbindung der Formel:

I°-O-CO-CH-S-CO-$CH_3$
|
$CH_2COOH$

liefert, in welcher I° den Rest des Ionophors darstellt, dem eines seiner Hydroxyle fehlt, und daß die Thiolgruppe der obigen Verbindung durch Einwirkung des Hydroxylamins freigesetzt wird, um die Verbindung der Formel:

I°-O-CO-CH-SH
|
$CH_2COOH$

zu liefern, in welcher I° wie oben definiert ist.

11. Verfahren nach Anspruch 10, dadurch gekennzeichnet, daß I° Monensin oder Nigerizin, denen das primäre Hydroxyl fehlt, darstellt.

12. Anwendung der Konjugate nach einem der Ansprüche 1 bis 6 als Potenzierungsmittel von Immunotoxinen.

13. Pharmazeutische Komposition, dadurch gekennzeichnet, daß sie mindestens ein Immunotoxin und mindestens ein Konjugat nach einem der Ansprüche 1 bis 6 umfaßt.

14. Aktivierte Carboxylionophore, die eine gegebenenfalls acetylierte Thiolgruppe enthalten, welche künstlich durch Umsetzung mit S-Acetylmercaptobernsteinsäureanhydrid und gegebenenfalls Entfernung der Acetylgruppe mittels Hydroxylamin eingeführt wurde.

15. Aktivierte Carboxylionophore nach Anspruch 14 der Formel:

I°-O-CO-CH-S-W
|
$CH_2COOH$

worin I° den Rest eines Ionophors darstellt, dem eines seiner Hydroxyle fehlt, und W für Wasserstoff oder eine Acetylgruppe steht.

16. Aktivierte Carboxylionophore nach Anspruch 15 der Formel:

I°-O-CO-CH-S-W
|
$CH_2COOH$

worin I° Monensin oder Nigerizin, denen ihr primäres Hydroxyl fehlt, darstellt.

**Patentansprüche** für den Vertragsstaat: AT

1. Verfahren zur Herstellung eines Konjugats zwischen einem monovalenten Carboxylionophor und einem Makromolekül, dadurch gekennzeichnet, daß es in der Kopplung mittels kovalenter Bindung des monovalenten Carboxylionophors mit einem Makromolekül, ausgewählt aus Antikörpern, Antikörperfragmenten, Proteinen, wie Peptidhormonen oder Humanproteinen, Peptidliganden und durch kovalente Kopplung eines Antikörpers und eines Proteins erhaltenen gemischten Makromolekülen, besteht.

2. Verfahren nach Anspruch 1, dadurch gekennzeichnet, daß das Makromolekül ein monoklonaler Antikörper ist.

3. Verfahren nach einem der Ansprüche 1 oder 2, dadurch gekennzeichnet, daß die Kopplung mittels kovalenter Bindung durch eine Disulfidbindung oder eine Thioetherbindung erzielt ist.

4. Verfahren nach einem der Ansprüche 1 bis 3, dadurch gekennzeichnet, daß das Ionophor eine primäre Hydroxylgruppe besitzt.

5. Verfahren nach einem der Ansprüche 1 bis 4, dadurch gekennzeichnet, daß das Ionophor Monensin, Nigerizin, Grisonixin und Lasalozid ist.

6. Verfahren nach einem der Ansprüche 1 bis 5, dadurch gekennzeichnet, daß das Makromolekül ausgewählt ist aus dem Antikörper T 29-33, dem Antikörper 3E10, dem Antikörper anti-DNP und menschlichem Serumalbumin.

7. Verfahren nach einem der Ansprüche 1 bis 6, dadurch gekennzeichnet, daß es darin besteht, daß:

1) eine Thiolgruppe in eine der zu koppelnden Verbindungen eingeführt wird;

2) eine oder mehrere mit den Thiolfunktionen reagierfähige Funktionen in die andere Verbindung eingeführt

werden;

3) die beiden so erhaltenen Verbindungen in wässerigem Milieu bei einem pH zwischen 5 und 9 und einer Temperatur von unter 30°C reagieren gelassen werden.

8. Verfahren nach Anspruch 7, dadurch gekennzeichnet, daß man das eine Thiolgruppe tragende Ionophor mit einem Makromolekül reagieren läßt, in welches man eine mit der Thiolfunktion reagierfähige Gruppe eingeführt hat, welche Gruppe die Formel -S-S-X aufweist, worin X eine Aktivatorgruppe darstellt.

9. Verfahren nach Anspruch 7, dadurch gekennzeichnet, daß man das eine Thiolgruppe tragende Ionophor mit einem Makromolekül reagieren läßt, in welches man eine mit der Thiolfunktion reagierfähige Gruppe eingeführt hat, welche Gruppe die Formel:

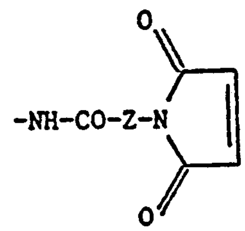

aufweist, worin Z eine aliphatische oder aromatische Raumstruktur mit 1 bis 10 Kohlenstoffatomen darstellt.

10. Verfahren nach Anspruch 9, dadurch gekennzeichnet, daß die Einführung der Thiolgruppe in das Ionophor durch Umsetzung des Ionophors mit dem S-Acetyl-mercaptobernsteinsäureanhydrid erfolgt und eine Verbindung der Formel

$$I°\text{-O-CO-}\underset{\displaystyle CH_2COOH}{\underset{|}{CH}}\text{-S-CO-}CH_3 \qquad \text{I}$$

liefert, in welcher I° den Rest des Ionophors darstellt, dem eines seiner Hydroxyle fehlt, und daß die Thiolgruppe der obigen Verbindung durch Einwirkung des Hydroxylamins freigesetzt wird, um die Verbindung der Formel:

$$I°\text{-O-CO-}\underset{\displaystyle CH_2COOH}{\underset{|}{CH}}\text{-SH}$$

zu liefern, in welcher I° wie oben definiert ist.

11. Verfahren nach Anspruch 10, dadurch gekennzeichnet, daß I° Monensin oder Nigerizin, denen das primäre Hydroxyl fehlt, darstellt.

**Claims** for the Contracting States: BE, CH, DE, FR, GB, IT, LI, LU, NL, SE

1. As novel products, the conjugates obtained by coupling by covalent bond of a monovalent carboxylic ionophore with a macromolecule, selected from the antibodies, antibody fragments, proteins such as peptide hormones or human proteins, peptide ligands and mixed macromolecules obtained by covalent coupling of an antibody and a protein.

2. Conjugate according to claim 1, characterized in that the macromolecule is a monoclonal antibody.

3. Conjugate according to one of claims 1 or 2, characterized in that the coupling by means of a covalent bond is achieved by a disulfide bond or a thioether bond.

4. Conjugate according to any one of claims 1 to 3, characterized in that the ionophore has a primary hydroxyl group.

5. Conjugate according to any one of claims 1 to 4, characterized in that the ionophore is monensin, nigericin, grisonixin or lasalocid.

6. Conjugate according to any one of claims 1 to 5, characterized in that the macromolecule is selected from antibody T 29-33, antibody 3E10, antibody anti-DNP and human serum albumin.

7. Process for obtaining conjugates according to any one of claims 1 to 6, characterized in that it consists in:

1) introducing a thiol group into one of the compounds to be coupled;

2) introducing into the other compound one or more groups capable of reacting with the said thiol groups; and

3) reacting the two resulting compounds in an aqueous medium, at a pH of between 5 and 9 at a temperature below 30°C.

8. Process according to claim 7, characterized in that the ionophore carrying a thiol group is reacted with a macromolecule into which a group capable of reacting with the thiol group has been introduced, this reactive group having the formula -S-S-X, in which X represents an activating radical.

9. Process according to claim 7, characterized in that the ionophore carrying a thiol group is reacted with a macromolecule into which a group capable of reacting with the thiol group has been introduced, this reactive group having the formula:

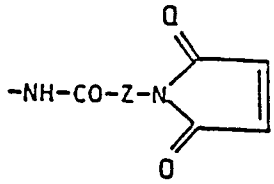

in which Z is an aliphatic or aromatic spacing structure containing from 1 to 10 carbon atoms.

10. Process according to claim 9, characterized in that the introduction of the thiol into the ionophore is carried out by reacting the ionophore with S-acetylmercapto-succinic anhydride to give a compound of the formula:

I°-O-CO-CH-S-CO-$CH_3$
|
$CH_2COOH$

in which I° represents the residue of the ionophore from which one of the hydroxyls has been removed, and wherein the thiol group of the above compound is freed by reaction with hydroxylamine to give the compound of the formula:

I°-O-CO-CH-SH
|
$CH_2COOH$

in which I° is as defined above.

11. Process according to claim 10, characterized in that I° is monensin or nigericin from which the primary hydroxyl has been removed.

12. Application of the conjugate according to any one of claims 1 to 6, as immunotoxins potentiating agents.

13. Medecine association, characterized in that it comprises at least an immunotoxin and at least a conjugate according to any one of claims 1 to 6.

14. Activated carboxylic ionophores containing a thiol group, optionally acetylated, artificially introduced by action of S-acetylmercaptosuccinic anhydride and optional elimination of the acetyl group by hydroxylamine.

15. Activated carboxylic ionophores according to claim 14, corresponding to the formula:

I°-O-CO-CH-S-W
|
$CH_2COOH$

in which I° represents the residue of an ionophore from which one of the hydroxyls has been removed, and W represents hydrogen or an acetyl group.

16. Activated carboxylic ionophores according to claim 15, having the formula:

I°-O-CO-CH-S-W
|
$CH_2COOH$

in which I° is monensine or nigericine from which the primary hydroxyls group has been removed.

**Claims** for the Contracting State: AT

1. Process for the obtention of a conjugate between a monovalent carboxylic ionophore and a macromolecule, characterized in that it consists in coupling the said monovalent carboxylic ionophore by means of a covalent bond with a macromolecule selected from the antibodies, antibody fragments, proteins such as peptide hormones or human proteins, peptide ligands and mixed macromolecules obtained by covalent coupling of an antibody and a protein.

2. Process according to claim 1, characterized in that the macromolecule is a monoclonal antibody.

3. Process according to one of claims 1 or 2, characterized in that the coupling by means of a covalent bond is achieved by a disulfide bond or a thioether bond.

4. Process according to any one of claims 1 to 3, characterized in that the ionophore has a primary hydroxyl group.

5. Process according to any one of claims 1 to 4, characterized in that the ionophore is monensin, nigericin, grisonixin or lasalocid.

6. Process according to any one of claims 1 to 5, characterized in that the macromolecule is selected from antibody T 29-33, antibody 3E10, antibody anti-DNP and human serum albumin.

7. Process according to any one of claims 1 to 6, characterized in that it consists in:
1) introducing a thiol group into one of the compounds to be coupled;
2) introducing into the other compound one or more groups capable of reacting with the said thiol groups; and
3) reacting the two resulting compounds in an aqueous medium, at a pH of between 5 and 9, at a temperature below 30°C.

8. Process according to claim 7, characterized in that the ionophore carrying a thiol group is reacted with a macromolecule into which a group capable of reacting with the thiol group has been introduced, this reactive group having the formula -S-S-X, in which X represents an activating radical.

9. Process according to claim 7, characterized in that the ionophore carrying a thiol group is reacted with a macromolecule into which a group capable of reacting with the thiol group has been introduced, this reactive group having the formula:

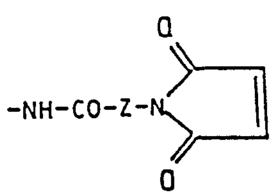

in which Z is an aliphatic or aromatic spacing structure containing from 1 to 10 carbon atoms.

10. Process according to claim 9, characterized in that the introduction of the thiol into the ionophore is carried out by reacting the ionophore with S-acetylmercaptosuccinic anhydride to give a compound of the formula:

I°-O-CO-CH-S-CO-$CH_3$
|
$CH_2COOH$

in which I° represents the residue of the ionophore from which one of the hydroxyls has been removed, and wherein the thiol group of the above compound is freed by reaction with hydroxylamine to give the compound of the formula:

I°-O-CO-CH-SH
|
$CH_2COOH$

in which I° is as defined above.

11. Process according to claim 10, characterized in that I° is monensin or nigericin from which the primary hydroxyl has been removed.

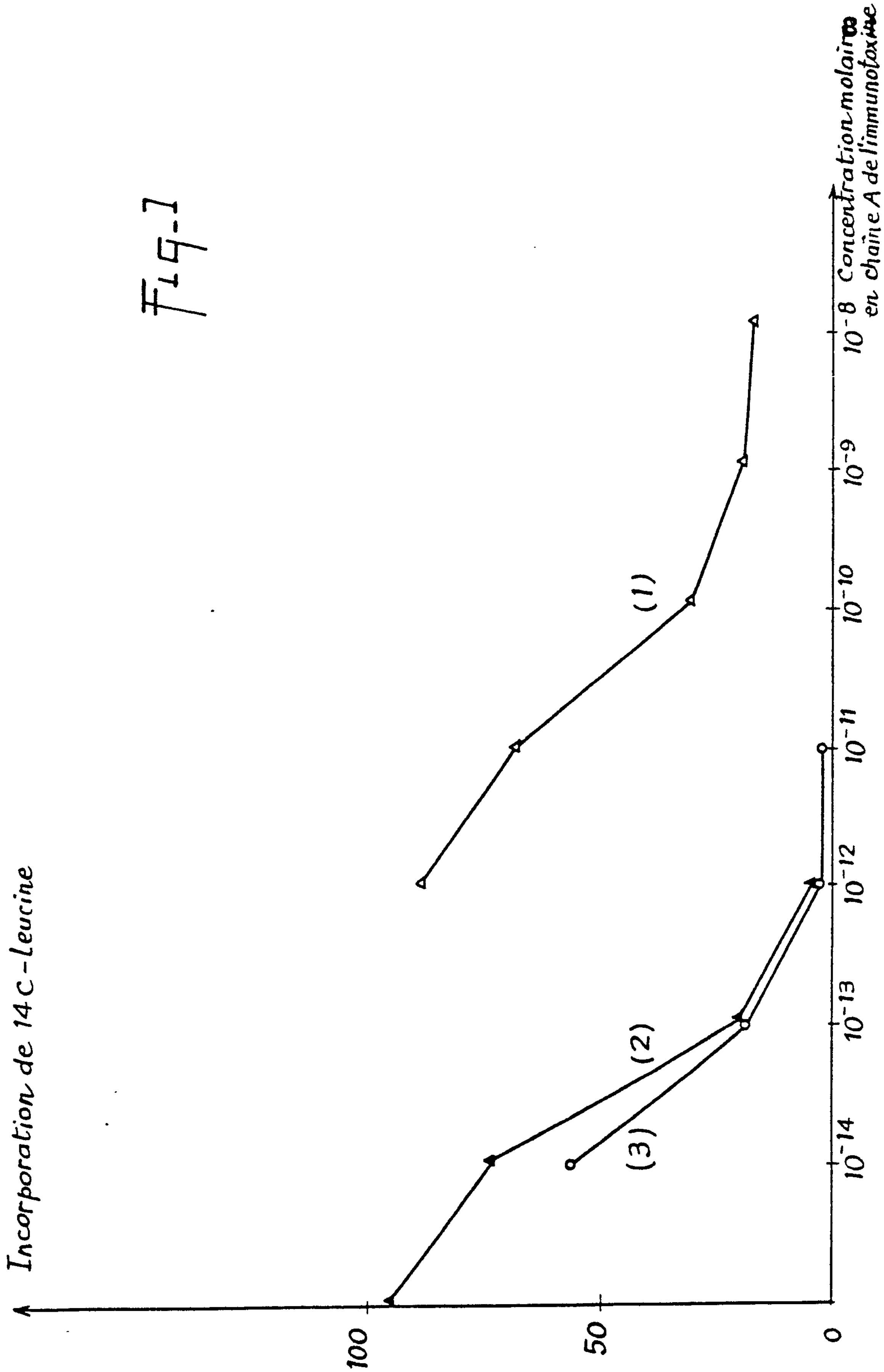
Incorporation de 14 C-Leucine
100
50
0
10^-14
10^-13
10^-12
10^-11
10^-10
10^-9
10^-8
concentration molaire en chaîne A de l'immunotoxine
(1)
(2)
(3)

Fig-1

Fig-2

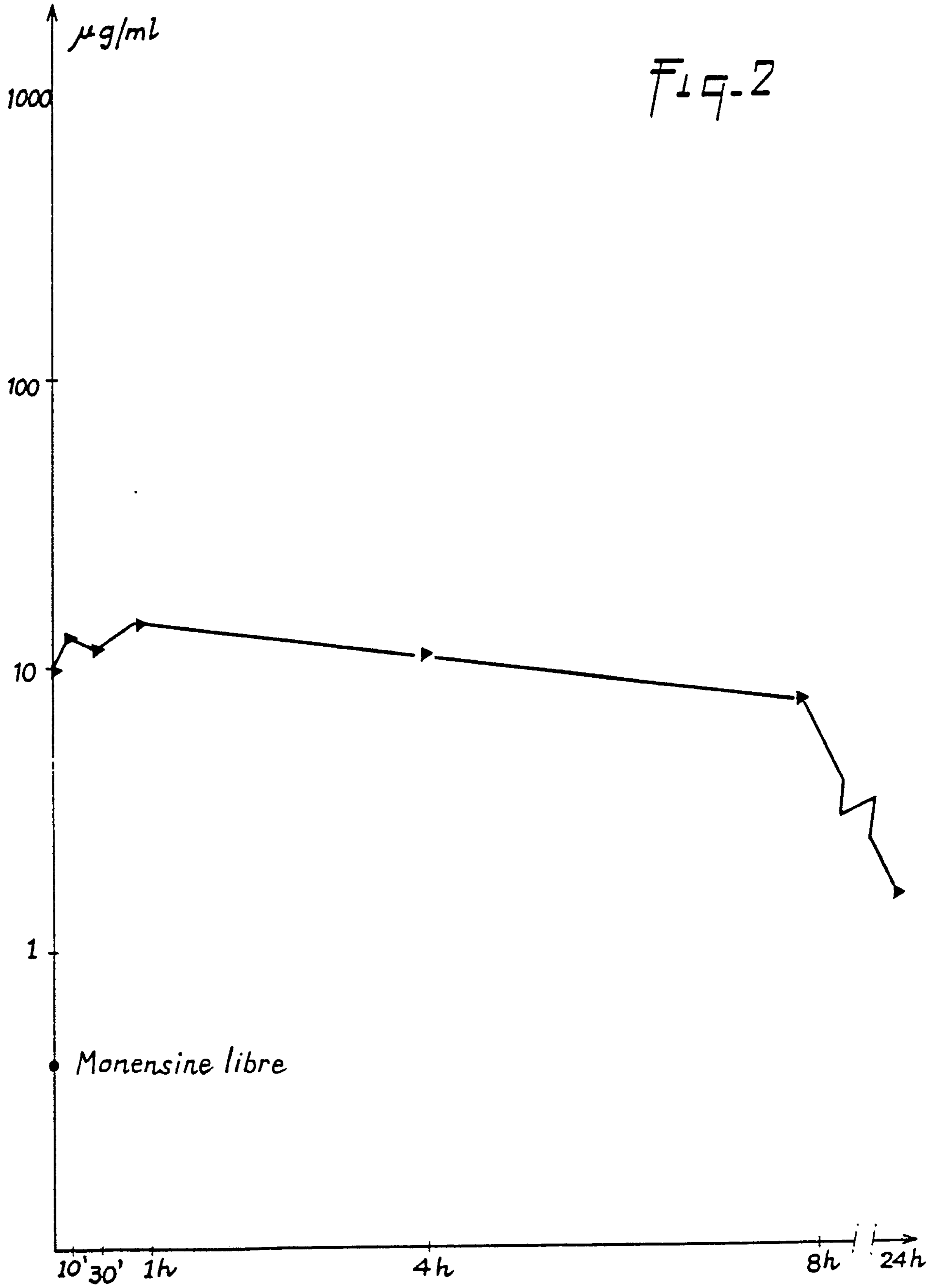

Fig-3

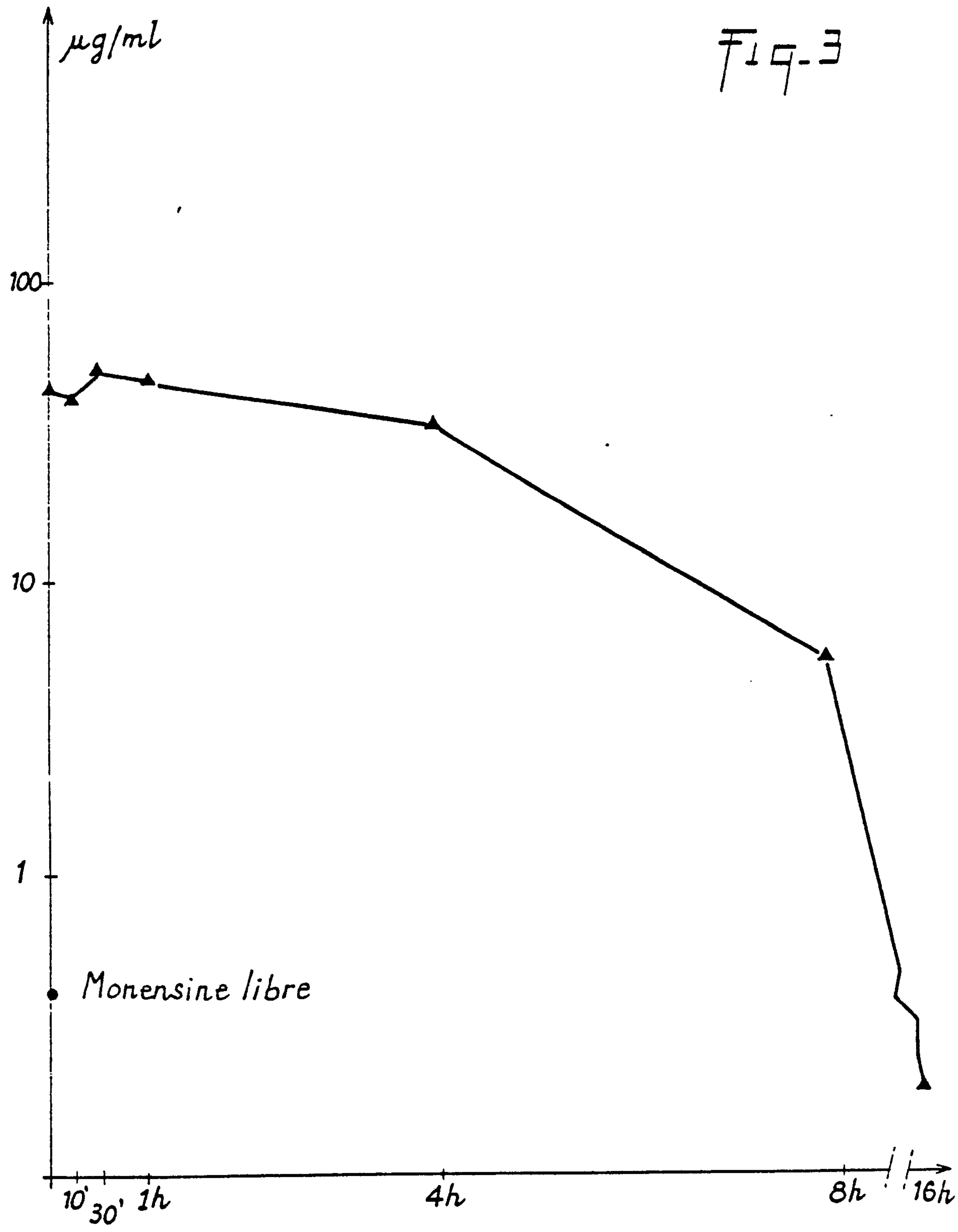

Fig-4

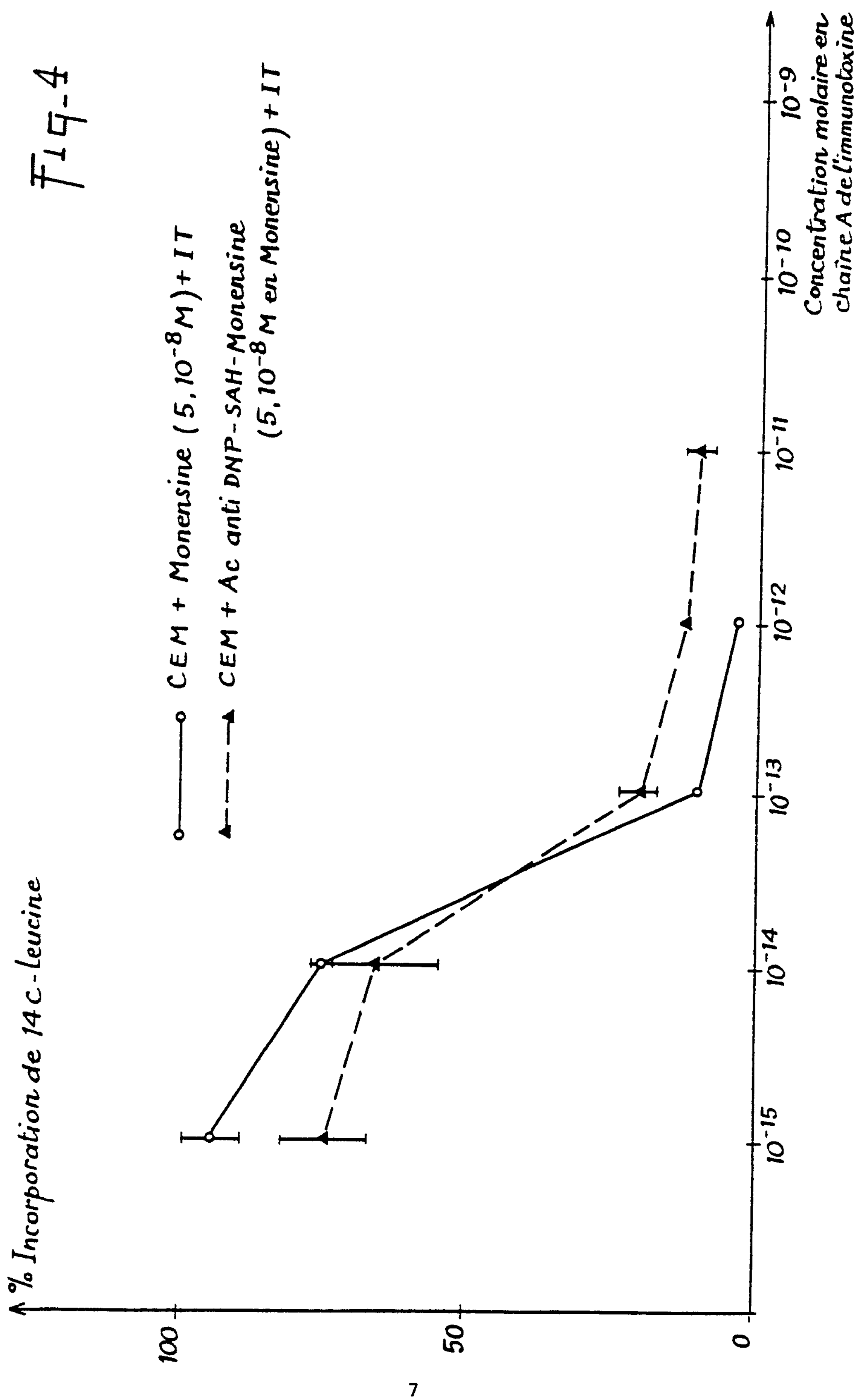
% Incorporation de 14C-leucine
CEM + Monensine (5,10$^{-8}$M) + IT
CEM + Ac anti DNP-SAH-Monensine (5,10$^{-8}$M en Monensine) + IT
100
50
0
$10^{-15}$
$10^{-14}$
$10^{-13}$
$10^{-12}$
$10^{-11}$
$10^{-10}$
$10^{-9}$
Concentration molaire en chaîne A de l'immunotoxine

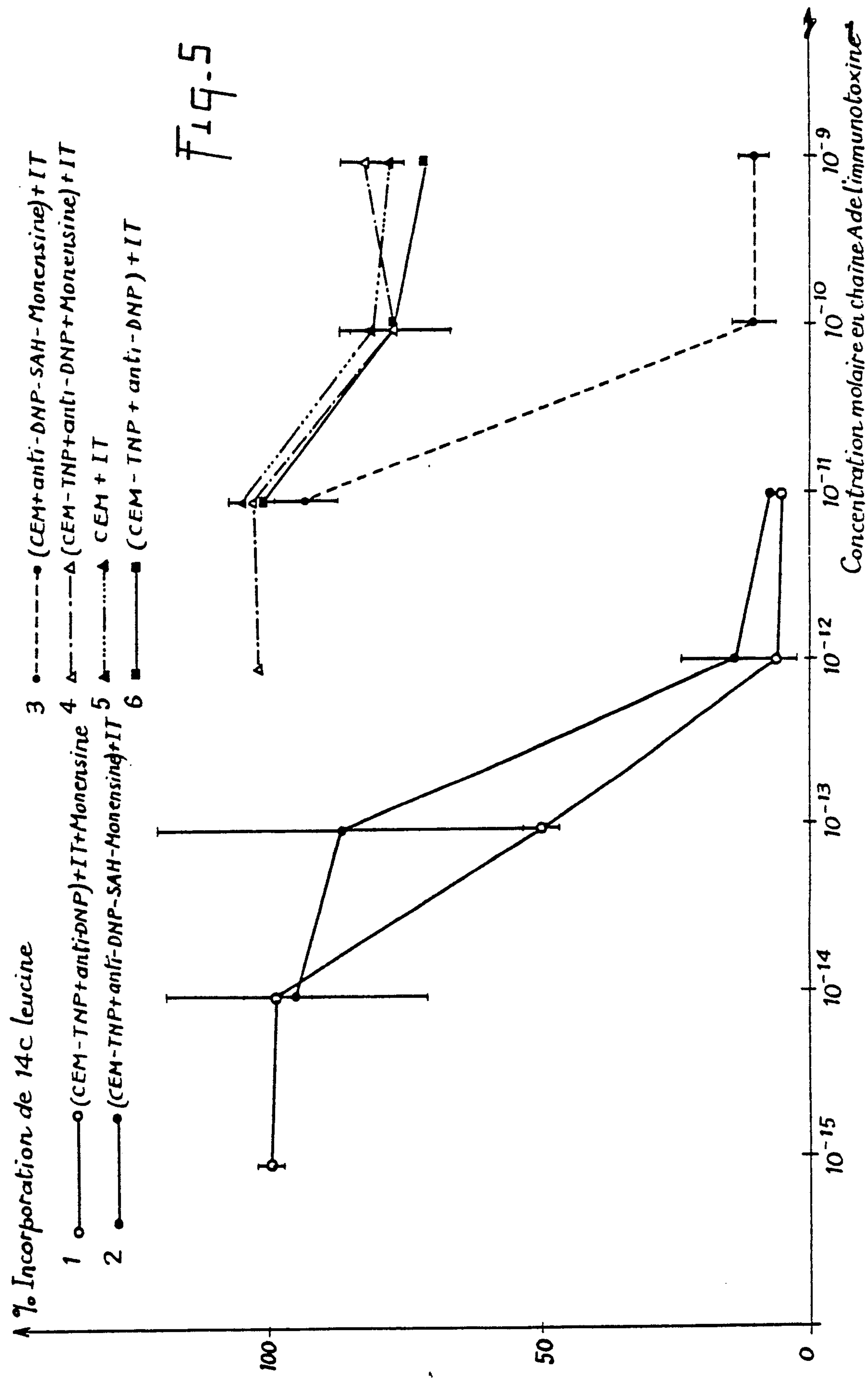
Fig-5
% Incorporation de 14c leucine
1 (CEM-TNP+anti-DNP)+IT+Monensine
2 (CEM-TNP+anti-DNP-SAH-Monensine)+IT
3 (CEM+anti-DNP-SAH-Monensine)+IT
4 (CEM-TNP+anti-DNP+Monensine)+IT
5 CEM + IT
6 (CEM - TNP + anti-DNP) + IT
100
50
0
10-15
10-14
10-13
10-12
10-11
10-10
10-9
Concentration molaire en chaîne A de l'immunotoxine